(19) Europäisches Patentamt

European Patent Office

Office européen des brevets

(11) **EP 1 025 273 B1**

(12) **FASCICULE DE BREVET EUROPEEN**

(45) Date de publication et mention
de la délivrance du brevet:
**05.12.2001 Bulletin 2001/49**

(21) Numéro de dépôt: **98929482.2**

(22) Date de dépôt: **04.06.1998**

(51) Int Cl.[7]: **C22C 38/38**, C22C 38/00

(86) Numéro de dépôt international:
**PCT/FR98/01130**

(87) Numéro de publication internationale:
**WO 98/55662 (10.12.1998 Gazette 1998/49)**

(54) **ACIER INOXYDABLE SANS NICKEL POUR APPLICATIONS BIOMEDICALES**

ROSTFREIES STAHL OHNE NICKEL FÜR BIOLOGISCH-MEDIZINISCHE VERWENDUNGEN

NICKEL-FREE STAINLESS STEEL FOR BIOMEDICAL APPLICATIONS

(84) Etats contractants désignés:
**AT CH DE FR GB IT LI LU SE**

(30) Priorité: **04.06.1997 FR 9706863**

(43) Date de publication de la demande:
**09.08.2000 Bulletin 2000/32**

(73) Titulaire: **SOCIETE INDUSTRIELLE DE METALLURGIE AVANCEE S.I.M.A. Société Anonyme F-92200 Neuilly sur Seine (FR)**

(72) Inventeurs:
• **MONTAGNON, Jacques**
**F-63000 Clermont Ferrand (FR)**

• **MORAUX, Jean-Yves**
**F-75017 Paris (FR)**

(74) Mandataire: **Busnel, Jean-Benoît et al Cabinet Beau de Loménie, 158, rue de l'Université 75340 Paris Cédex 07 (FR)**

(56) Documents cités:
**EP-A- 0 422 360      WO-A-91/16469**
**CH-A- 684 979        DE-U- 9 215 141**
**FR-A- 2 071 667      GB-A- 506 905**

## EP 1 025 273 B1

**Description**

L'invention concerne une classe d'aciers inoxydables austénitiques sans nickel, résistant à la corrosion dans les milieux physiologiques, capable de satisfaire toutes les valeurs minimales des caractéristiques et propriétés spécifiées dans la norme ISO 5832-1 "Aciers à forger inoxydables pour implants chirurgicaux", et pouvant être élaborée dans les fours conventionnels sans dispositif particulier de mise en pression du four.

La très faible teneur résiduelle en nickel de cette classe d'acier permet de se prémunir de réactions allergiques à cet élément pour des applications internes et/ou externes au corps humain.

Le W091/16469 divulgue un acier inoxydable austénitique amagnétique ayant à la fois des propriétés mécaniques et une bonne résistance à la corrosion, en particulier, sous contrainte. Cet acier a une composition qui est adaptée à une utilisation dans des équipements de forage et qui ne se préoccupe pas, par conséquent, des problèmes de relargage de nickel dans les milieux physiologiques.

Or, dans le domaine des biomatériaux, la législation de nombreux pays s'oriente actuellement vers la limitation, voire l'élimination du nickel des alliages en contact avec la peau ou utilisés pour la fabrication de matériel prothétique temporaire (reconstruction osseuse) ou permanent (prothèse). En effet, il est prouvé depuis plusieurs années que les relargages d'ions nickel de certains biomatériaux dans le corps humain peuvent conduire à des irritations locales et même dans certains cas, à des infections. Lors de tests intracutanés, il a été mis en évidence que 15% de la population serait susceptible de réactions allergiques au nickel.

Parmi les matériaux concernés, les aciers inoxydables austénitiques alliés au nickel sont particulièrement concernés eu égard à leur large utilisation pour la réalisation de pièces implantées ou en simple contact avec le corps humain, telles que :

- les implants osseux temporaires ou permanents,
- les fixateurs externes de réduction des fractures,
- les montres et bracelets de montres.

L'emploi de ces aciers alliés au nickel s'est jusqu'à présent imposé en raison de l'excellent compromis entre leurs diverses caractéristiques, telles que résistance mécanique intrinsèque, tenue à diverses formes de corrosion et résistance mécanique de leur surface, par exemple en condition de frottement.

Le domaine des implants, qui est actuellement le plus critique vis-à-vis des risques allergiques, a servi de base à l'étude de l'acier objet de la présente invention.

En effet, dans ce domaine, le recours à d'autres alliages que les aciers se heurte à des insuffisances techniques ou à des problèmes économiques liés au coût de ces matériaux ou à leur mise en oeuvre.

Selon les recommandations du système ISO, des aciers austénitiques peuvent être utilisés actuellement pour la réalisation d'implants à destination humaine; ces aciers sont définis par les normes ISO 5832/1 et 5832/9. Leur teneur en nickel doit être comprise entre 9 et 15 % et leur utilisation est donc susceptible de conduire à des relargages de nickel, même en l'absence de développement d'un quelconque mécanisme de corrosion.

Le film passif qui les recouvre et qui leur confère leurs propriétés de tenue à la corrosion peut relarguer des ions nickel sous l'effet d'arrachements mécaniques en conditions de frottement ou sous l'effet des équilibres électrochimiques avec le milieu environnant, qui impliquent les lentes dissolution et reconstruction simultanées de ce film superficiel.

D'autre part, les aciers inoxydables austénitiques au nickel sont reconnus pour leur sensibilité aux mécanismes de corrosion sous contrainte ou de fatigue-corrosion lorsqu'ils sont soumis à des contraintes qui dépassent un certain "seuil" en relation avec la mise en plasticité de micro-domaines en contact avec le milieu corrosif : le nickel joue un rôle dans le développement de ces mécanismes de corrosion qui sont particulièrement néfastes puisqu'ils peuvent conduire à la rupture de la pièce.

Les aciers ferritiques, sans nickel et plus résistants à la corrosion sous contrainte, ne peuvent pas être utilisés en tant qu'implants humains du fait de leurs propriétés ferro-magnétiques.

Le chrome, qui confère l'inoxydabilité aux aciers au-delà d'une teneur minimale de 13% environ, et le molybdène qui complète et stabilise fortement l'inoxydabilité des aciers au chrome, doivent, selon la norme ISO 5832-1 être additionnés à des teneurs capables de répondre à la relation suivante, de façon a satisfaire la tenue à la corrosion en milieux physiologiques :

(% Cr) + 3,3 (% Mo) $\geq$ 26, exprimée en concentration pondérale des éléments d'alliage.

Outre le chrome et le molybdène, les aciers inoxydables austénitiques au manganèse contiennent des proportions variables en manganèse et azote qui modifient leur degré d'inoxydabilité : le manganèse, moins noble que le nickel dans l'échelle des potentiels d'oxydo-réduction, affecte sensiblement la tenue à la corrosion des aciers inoxydables, tandis que l'azote est réputé l'améliorer.

Des études récentes tendent à montrer que l'on peut relier, en première approximation, la tenue à la corrosion en milieux chlorurés des aciers inoxydables au manganèse à leur composition en principaux éléments d'alliages selon la relation suivante :

(%Cr) + 3.3 (%Mo) + 30 (%N) - (%Mn) dont la valeur est caractéristique de la tenue à la corrosion desdits aciers.

Ainsi, plus sa valeur est élevée, meilleure est la tenue à la corrosion de l'acier.

De préférence, la proportion en éléments d'alliage doit être telle que :

$$(\%Cr) + 3,3\,(\%Mo) + 30\,(\%N) - (\%Mn) \geq 26$$

de façon à obtenir une résistance à la corrosion dans les milieux de l'application envisagée au moins équivalente à celle de la nuance D décrite dans la norme ISO 5832-1.

Les aciers inoxydables austénitiques au manganèse peuvent contenir du carbone dans le but de stabiliser la structure austénitique. Toutefois, l'addition de carbone conduit à une dégradation très importante de la tenue à la corrosion, dès les valeurs aussi basses que 0,1% en poids. En effet, le carbone forme des carbures avec les éléments chrome et molybdène en particulier, ce qui a pour conséquence d'appauvrir la matrice austénitique située autour des carbures en ces éléments Cr et Mo qui confèrent néanmoins l'inoxydabilité. Il pourra alors se développer des formes de corrosion localisées autour des carbures, telle que la corrosion intergranulaire. En outre, le carbone est nocif à la tenue à la corrosion sous contrainte.

Dans le cas précis d'application en milieux physiologiques, la teneur en carbone d'un acier inoxydable doit rester inférieure à 0,1 % en poids, préférentiellement inférieure à 0,06%.

Compte tenu des problèmes techniques précédents liés aux aciers connus, il existe donc actuellement une forte demande que se propose de satisfaire l'invention, pour des aciers inoxydables austénitiques amagnétiques à très basse teneur en nickel et ayant les propriétés suivantes :

- absence de ferro-magnétisme,
- niveau de résistance mécanique égal ou supérieur à ceux des aciers existants dans les normes ISO 5832/1 et 5832/9, sans fragilité,
- tenue à la corrosion en milieux physiologiques sensiblement égale ou supérieure à celle de l'acier défini par l'ISO 5832/1,
- bonne aptitude au polissage,
- bonne tenue en fatigue,
- meilleure résistance aux formes de corrosion sous contrainte en milieux physiologiques jusqu'à des contraintes proches de leur limite élastique conventionnelle à 0,2%,
- très faibles teneurs en inclusions et éléments résiduels indésirables,
- coûts de fabrication équivalents ou plus faibles que ceux des aciers austénitiques traditionnels en particulier par la recherche d'une composition d'alliage spécifique susceptible d'être élaborée par des moyens conventionnels sans étape de refusion sous haute pression d'azote,
- grandes facilités de durcissement par écrouissage mécanique.

Ce but est atteint, selon l'invention au moyen d'un acier dont la composition en pourcentages pondéraux est la suivante :

- de 15 à 20 % de Cr
- de 2,5 à 4% de Mo
- de 15 à 24 % de Mn
- de 0,6 à 0,85 % de N
- de 0,1 à 0,5 % de V
- de 0 à 0,25% de Ni
- de 0 à 0,06% de C
- de 0 à 0,25% de Si
- de 0 à 0,02 % de B
- de 0 à 0,5% de Nb + Ta
- de 0 à 0,5 % de Co
- de 0 à 0,5% de Cu
- de 0 à 0,002% de S
- de 0 à 0,02 % de Ti
- de 0 à 0,02% de Al.

le reste étant constitué de fer et d'impuretés, avec les conditions suivantes :

$$(\%Cr) + 2,5\,(\%Mo) \leq \text{à } 27 \tag{I}$$

$$(\%Cr) + 3,3\,(\%Mo) \geq \text{à } 26, \tag{II}$$

et

$$LOG\,(\%N) + 0,0605\,(\%N) = -1,3 + [125\,(\%V) + 80\,(\%Nb) + 52$$

$$(\%Cr) + 19\,(\%Mn)] \times 10^{-3} - [4,3\,(\%Cr)^2 + 0,35\,(\%Mn)^2] \times 10^{-4} +$$

$$0,17\,(\%Cr)^3 \times 10^{-5}. \tag{III}$$

Selon une composition préférentielle,

$$(\%Cr) + 3,3\,(\%Mo) + 30\,(\%N) - (\%Mn) \text{ est supérieure ou égale à } 26 \tag{IV}$$

Selon une caractéristique avantageuse, la limite élastique conventionnelle à l'essai de traction Rp 0,2 est supérieure ou égale à 450 N/mm² après mise en solution à une température supérieure ou égale à 1000°C et hypertrempe.

Selon une autre caractéristique, la limite élastique conventionnelle à l'essai de traction Rp 0,2 est supérieure ou égale à 1000 N/mm² et l'allongement à la rupture A5d est supérieur à 25% après écrouissage à température ambiante du métal traité par mise en solution à une température supérieure ou égale à 1000°C et hypertrempe.

Un autre objet de l'invention est une utilisation de l'acier précédent pour la réalisation de pièces destinées à être en contact avec le corps humain et notamment les tissus en application interne ou externe.

L'acier de la présente invention est constitué d'une structure purement austénitique après un traitement de mise en solution au-delà de 1000°C, alors même que sa composition ne comporte pas d'addition d'éléments carbone et nickel habituellement utilisés en proportion variable pour stabiliser l'austénite des aciers riches en chrome et molybdène. Les valeurs maximales des teneurs en carbone et nickel de l'acier de la présente invention sont donc respectivement de 0,06% et 0,25%, ceci dans les buts précis de produire un acier résistant aux phénomènes de corrosion intergranulaire et de corrosion sous tension et d'éliminer les relargages d'ions nickel à partir du film passif qui le recouvre, lorsqu'il est utilisé comme bio-matériau, en contact avec des tissus humains.

Une particularité de l'acier de l'invention est que sa structure austénite est exclusivement stabilisée par les éléments gammagènes azote, manganèse et, éventuellement, le bore, en l'absence de nickel et de carbone qui sont alors considérés comme des impuretés résiduelles. Les éléments gammagènes favorisent et/ou stabilisent la phase cristallographique cubique face centrée de l'austénite gamma. Les impuretés sont inévitables avec les moyens d'élaboration utilisés tels que le four à arc, le réacteur AOD (convertisseur Argon, Oxygène, décarburation) ou les procédés standards de refusion sous laitier, sans dispositif particulier de mise en pression d'azote du four d'élaboration.

En particulier, les éléments cobalt et cuivre, qui sont aussi des stabilisateurs de l'austénite et que l'on retrouve comme impuretés en quantité variable dans les matières premières nécessaires à l'élaboration des aciers inoxydables, seront strictement limités à une teneur maximale de 0.5% chacun, pour les raisons suivantes :

- le cobalt est un élément métallique qui a été cité parmi ceux présentant une nocivité pour le corps humain,
- le cuivre est un élément auquel on reconnaît plusieurs avantages lorsqu'il est ajouté dans les aciers inoxydables, comme une amélioration à l'aptitude à l'usinage et de la tenue à la corrosion. Toutefois, l'art antérieur ne prévoyait pas que des additions de cuivre pouvaient dégrader irrémédiablement la forgeabilité des aciers inoxydables à forte teneur en manganèse tels que ceux de la présente invention, lorsqu'ils sont moulés en lingotières, à la suite de la formation par ségrégation dans les lingots d'un composé à base de manganèse et de cuivre et à très bas point de fusion.

Enfin, le cuivre et le cobalt limitent fortement la solubilité en azote dans l'acier liquide lors d'une élaboration avec les moyens conventionnels, sans dispositif de mise en pression du four d'élaboration ; or, l'azote est indispensable pour atteindre les propriétés requises.

Pour les raisons indiquées ci-dessus, l'acier de l'invention a des teneurs en cobalt et en cuivre strictement contrôlées à moins de 0,5% chacune.

Une autre particularité importante de l'acier de l'invention est que les teneurs en éléments chrome et molybdène doivent être telles que (Cr%) + 2,5 (Mo%) ≤ 27. En effet, en utilisant des moyens classiques d'élaboration, sans dispositif de mise sous pression du four et en imposant une pression partielle saturante en azote dans le métal liquide, l'acier de la présente invention est purement austénitique lorsque sa composition satisfait au moins la condition ci-dessus.

Les rôles spécifiques de chacun des éléments d'addition majeurs de l'acier de l'invention peuvent être précisés de la façon suivante

- le chrome est l'élément prépondérant pour la construction du film passif qui protège le métal de la corrosion. Malgré sa tendance alphagène intrinsèque, son rôle est aussi prépondérant dans l'obtention d'un acier austénitique puisqu'il favorise très fortement la solubilité de l'azote, en particulier dans l'acier liquide.

Les éléments alphagènes favorisent et/ou stabilisent la phase cristallographique cubique centrée de la ferrite alpha stable à basse température et de la ferrite delta stable à haute température.

Etant donné le rôle sigmagène du chrome, sa teneur est préférablement limitée à 20%. Les éléments sigmagènes sont ceux qui favorisent et/ou stabilisent la phase sigma qui est un composé intermétallique ordonné du système Fe-Cr-Mo ayant une très grande dureté.

Le molybdène participe fortement à la résistance aux corrosions localisées en milieux chlorurés réducteurs ; à ce titre, son addition à des teneurs les plus élevées possibles est indispensable pour les applications biomédicales. Toutefois, cet élément est à la fois très alphagène et sigmagène et relève aussi la température de mise en solution de la phase sigma. Contrairement au chrome, il favorise peu la solubilité de l'azote et, enfin, il a une tendance marquée à ségréger dans le lingot. Pour ces raisons, la teneur maximale en molybdène est limitée à 4%.

Le manganèse est l'un des éléments qui stabilise directement l'austénite. De plus, il accroît la solubilité de l'azote dans l'acier liquide, mais dans une moindre proportion que le chrome. Enfin, associé à l'azote, il favorise nettement le durcissement de l'austénite lors d'opération d'écrouissage à froid. Toutefois, sa teneur maximale est fixée à 24% du fait de sa tendance à fragiliser l'acier aux températures de forgeage par formation de phases intermétalliques, et pour son influence défavorable à haute concentration sur la tenue à la corrosion des aciers inoxydables.

L'azote est le principal élément stabilisateur de l'austénite ; à ce titre, sa teneur doit être d'au moins 0,6%. Cet élément permet aussi d'accroître la tenue à la corrosion et la résistance mécanique de l'acier. Des additions excessives d'azote sont à proscrire de façon à éviter la formation de "soufflures" à la solidification des lingots ainsi que la précipitation de nitrures aux températures de mise en solution.

D'autre part, la teneur en azote est limitée à la valeur maximale de 0,85% car des teneurs supérieures induisent une fragilisation de l'austénite. en particulier lorsqu'un durcissement par écrouissage est réalisé car la température de la transition ductile/fragile croît en proportion de la teneur en azote et devient proche du domaine des températures d'utilisation.

La teneur maximale en azote est naturellement donnée par sa solubilité dans l'acier liquide, à température et pression données, L'acier de l'invention comporte des additions d'azote dans des proportions qui permettent d'une part son élaboration à la pression atmosphérique avec des moyens conventionnels (four à arc, AOD, four de refusion sous laitier) et, d'autre part, la stabilisation effective de l'austénite,

Ces propriétés spécifiques sont obtenues lorsque la teneur en azote (N) dans l'acier de l'invention satisfait la relation :

$$\text{LOG} (\%N) + 0,0605 (\%N) = -1,3 + [125 (\%V) + 80 (\%Nb) + 52$$

$$(\%Cr) + 19 (\%Mn)] \times 10^{-3} - [4,3 (\%Cr)^2 + 0,35 (\%Mn)^2] \times 10^{-4} +$$

$$0,17 (\%Cr)^3 \times 10^{-5}.$$

L'addition de vanadium est une particularité de l'acier de l'invention. Cet élément accroît la solubilité de l'azote dans l'acier liquide lors de son élaboration, ce qui permet d'ajuster la teneur en azote de l'acier à des valeurs élevées, dans les limites indiquées. Toutefois, des teneurs croissantes en vanadium provoquent l'extension du domaine de stabilité du nitrure de vanadium et, en particulier, relèvent sa température de solvus. De façon à conserver un acier purement austénitique après traitement préconisé de mise en solution et hypertempé, la teneur maximale en vanadium est limitée à 0,5%.

Le bore stabilise fortement l'austénite. En revanche, sa solubilité limitée dans l'acier de l'invention ne permet pas d'envisager des additions conséquentes. Sa teneur est donc limitée à 200ppm,

Parce qu'ils sont susceptibles de modifier les équilibres de phase, mais aussi parce qu'ils créent des composés durs qui ne permettent pas d'obtenir un parfait état de surface par polissage de l'acier, les éléments carburigènes niobium et tantale doivent être limités au strict minimum nécessaire aux caractéristiques qu'apportent ces éléments, telle la stabilisation du carbone de l'acier.

Parmi les éléments qui sont des impuretés résiduelles dans l'acier, certains doivent être strictement contrôlés et limités à des teneurs très basses car ils dégradent les propriétés recherchées.

La teneur en soufre sera limitée à 20 ppm dans les buts précis d'éviter la fragilité intergranulaire que provoque cet élément à toute température, et en particulier aux températures de forgeage, mais aussi pour diminuer au mieux la teneur en inclusions non métalliques que forme cet élément, en particulier avec le manganèse.

Le silicium favorable pour certains aspects tels que la désoxydation du métal liquide, sera contrôlé à une teneur inférieure ou égale à 0,25% car cet élément est un puissant formateur de la phase sigma fragilisante et nuisible à la tenue à la corrosion en général. C'est aussi un élément qui favorise les ségrégations dans les lingots, ce qui est un phénomène défavorable.

Les éléments titane et aluminium doivent être strictement contrôlés de façon à éviter, ou limiter, la formation de leurs nitrures très peu solubles, qui fixent inutilement une fraction d'azote sous forme de composés durs et fragilisants. Leur teneur est limitée à 0,02% maximum.

L'acier selon l'invention possède les propriétés requises à la suite d'un traitement thermique de mise en solution et d'hypertrempe depuis une température supérieure à 1000C pour des dimensions au moins égales à celles des produits fabriqués pour les applications envisagées.

Sa structure est purement austénitique et non ferro-magnétique,

Après traitement, selon les modalités définies ci-dessus, ses caractéristiques minimales sont les suivantes :
résistance maximale de la traction :          $Rm \geq 800$ N/mm$^2$,
limite élastique conventionnelle à 0,2 % :          $Rp0,2 \geq 450$ N/mm$^2$,
allongement à la rupture (la base de mesure étant de 5 fois le diamètre de l'éprouvette :          $A5d \geq 40\%$.

En outre, sa ténacité est excellente.

Après écrouissage à froid de barres, la limite élastique à l'essai Rp0,2 peut atteindre 1100 N/mm$^2$ tandis que l'allongement à la rupture reste supérieur à 25%, ce qui excède les caractéristiques des aciers définis dans la norme ISO 5832-1.

Pour une limite élastique de l'acier écroui de 1300 N/mm$^2$, l'allongement à rupture reste supérieur ou égal à 10%.

Sa tenue à la corrosion a été appréciée à l'aide d'un test électrochimique spécifique des conditions extrêmes rencontrées en cas de corrosion localisée par crevasse en milieux chlorurés ; le milieu d'essai est constitué d'une solution aqueuse à 2 moles de chlorure de sodium et est acidifiée à pH 1,2 par ajout d'acide chlorhydrique.

Le tableau ci-après donne les résultats de ce test sur deux coulées A et B d'aciers de l'invention, comparativement à une coulée C répondant au critère (% Cr) + 3,3 (% Mo) $\geq$ 26 et qui reprend la composition de l'invention à l'exception du fait que
     (% Cr) + 3,3 (% Mo) + 30 (%N) - (%Mn) est inférieur à 26, et comparativement à, d'une part, une coulée D d'acier austénitique au manganèse de l'art antérieur qui ne satisfait pas les deux conditions ci-dessus, et d'autre part, une coulée E d'un acier tel que décrit dans la norme ISO 5832-1, et dont la tenue à la corrosion sert de référence.

La densité des courants de dissolution mesurée au maximum du pic d'activité donne une notion de la vitesse de corrosion du métal dans le milieu considéré, c'est-à-dire que plus la densité de courant est élevée et plus le métal se corrode rapidement. La valeur du potentiel de rupture traduit quant à elle la stabilité de la passivité du métal ; plus ce potentiel est élevé et plus le métal résistera à l'amorçage d'un mécanisme de corrosion localisée. Les résultats des essais donnés dans le tableau suivant montrent :

a) la meilleure résistance à l'initiation des mécanismes de corrosion localisée des coulées A et B de l'acier de l'invention, toutes deux ayant un potentiel de rupture supérieur à celui de l'acier E selon ISO 5832-1, et qui conserve des valeurs élevées même dans des conditions de forte acidification du milieu ;
b) une tenue à la corrosion active en milieu réducteur acide sensiblement équivalente de la coulée B de l'invention et de la coulée E (ISO 5832-1) ;
c) une activité légèrement plus élevée de la coulée A de l'invention comparativement à la coulée B : cet effet est attribuable à la moindre teneur en molybdène de la coulée A, teneur qui est très proche de la valeur minimale spécifiée pour l'acier de l'invention ;
d) une bonne adéquation de la relation
     (% Cr) + 3,3 (% Mo) + 30 (% N) - (% Mn) avec la tenue à la corrosion des aciers inoxydables alliés au manganèse ; un classement de la tenue à la corrosion des aciers dans les solutions chlorurées telles que le milieu physiologique est plus précis avec la relation ci-dessus que lorsqu'on utilise la relation classique (% Cr) + 3,3 (% Mo) car la première fait apparaître le rôle très bénéfique de l'azote et le rôle néfaste du manganèse ;

e) les coulées C et D qui ne satisfont pas l'ensemble des prescriptions caractéristiques de la composition de l'invention, présentent des critères de tenue à la corrosion inférieurs à la coulée de référence E ; dans le cas de la coulée D, ce résultat est justifié par sa très faible teneur en molybdène tandis que dans le cas de la coulée C, la forte addition de molybdène n'est pas suffisante pour compenser la perte de résistance à la corrosion induite par les faibles teneurs en chrome et en azote et sa forte teneur en manganèse.

Il est admis que les courants d'échange métal/solution en l'absence de corrosion déclarée sont de l'ordre d'une dizaine de $\mu$A.cm$^{-2}$ pour les aciers inoxydables. Ceci signifie que les trois coulées A, B et E sont, dans la solution d'essai fortement chlorurée et acidifiée à pH 1,2, pratiquement dans un état passif et que cet état passif serait assuré par un pH peu supérieur.

Toutefois, une telle acidité ne peut pas être rencontrée à l'état naturel dans le corps humain. Ce n'est qu'après le développement d'une corrosion localisée (type crevasse) que le milieu environnant est peu à peu acidifié par sa réaction avec les produits de corrosion.

Or, il est aussi admis que le rôle bénéfique essentiel de l'azote dans les aciers inoxydables vis-à-vis de leur comportement en corrosion localisée est justement de neutraliser significativement cet effet par une réaction chimique.

En effet, l'azote libéré par le phénomène de corrosion entraîne la formation d'ions alcalins qui neutralise l'acidification locale.

On en déduit que, outre une bonne résistance dûment constatée à l'initiation de formes de corrosion localisée, les aciers de l'invention présentent vis-à-vis de la tenue à d'autres formes de corrosion, des comportements particulièrement favorables.

| Composition % pondéral | Coulée A | Coulée B | Coulée C | Coulée D | Coulée E (ISO 5832-1) |
|---|---|---|---|---|---|
| C | 0,037 | 0,052 | 0,045 | 0,035 | 0,012 |
| Si | 0,179 | 0,18 | 0,215 | 0,041 | 0,31 |
| Mn | 21,17 | 21,1 | 23,65 | 18,70 | 1,80 |
| Ni | 0,22 | 0,21 | 0,25 | 0,24 | 14,64 |
| Cr | 17,83 | 17,56 | 14,85 | 18,52 | 17,29 |
| Mo | 2,69 | 3 | 4,03 | 0,390 | 2,74 |
| N | 0,70 | 0,694 | 0,623 | 0,61 | 0,08 |
| V | 0,11 | 0,1 | 0,1 | 0,05 | - |
| B | - | - | - | - | - |
| Nb + Ta | < 0,020 | < 0,020 | < 0,010 | < 0,010 | - |
| Co | 0,33 | 0,031 | < 0,010 | 0,01 | 0,06 |
| Cu | 0,36 | 0,34 | 0,017 | 0,028 | 0,05 |
| S | 0,0013 | 0,0014 | 0,0019 | 0,0004 | < 0,002 |
| Ti | < 0,010 | <0,010 | < 0,010 | < 0,010 | - |
| Al | 0,016 | 0,009 | 0,018 | 0,0031 | - |
| Cr + 2,5 Mo | 24,55 | 25,05 | 24,92 | 19,5 | 24,14 |
| Cr + 3,3 Mo | 26,7 | 27,5 | 28,1 | 19,8 | 26,3 |
| Cr + 3,3 Mo + 30 N - Mn | 26,5 | 27,2 | 23,2 | 19,4 | 26,9 |
| i activité (1) ($\mu$A.cm$^{-2}$) | 45 | 20 | 460 | 10 000 | 15 |

(1) Densité des courants de dissolution mesurée au niveau maximum du pic d'activité lors de tests électrochimiques potentiocinétiques dans un milieu aqueux et chloruré (Na Cl - 2M) et acidifié à pH 1,2 par ajout d'acide chlorhydrique.

(suite)

| Composition % pondéral | Coulée A | Coulée B | Coulée C | Coulée D | Coulée E (ISO 5832-1) |
|---|---|---|---|---|---|
| E rupture (2) | > 500 m v/ECS | < 500 m V/ECS | 100 à 350 m V/ECS | < -100 m V/ECS | < 300 m V/ECS |

(2) Valeur de potentiel en balayage potentiocinétique anodique dans le milieu ci-dessus (1) à partir de laquelle les courants de dissolution croissent brutalement en relation avec l'amorçage de piqûres de corrosion.
Le potentiel est mesuré par rapport à une Electrode de référence à Calomel Saturée (ECS).

**Revendications**

1. Acier inoxydable amagnétique à hautes caractéristiques mécaniques, résistant à la corrosion dans les milieux physiologiques, et susceptible d'être élaboré à pression atmosphérique ayant la composition en % en poids suivante :

| | |
|---|---|
| Mn de 15 à 24 % | Ti $\leq$ 0,020 % |
| Cr de 15 à 20 % | Al $\leq$ 0,020 % |
| Mo de 2,5 à 4 % | S $\leq$ 0,0020 % |
| N de 0,6 à 0,85% | B $\leq$ 0,020 % |
| V de 0,1 à 0,5 % | Nb + Ta $\leq$ 0,5 % |
| C $\leq$ 0,06 % | Co $\leq$ 0,5 % |
| Ni $\leq$ 0,25 % | Cu $\leq$ 0,5 % |
| Si $\leq$ 0,25 % | |

et de 0 à 0,5 % en chacun des éléments Cu, Co et de la somme Nb + Ta, le reste étant constitué de fer et d'impuretés et en ce que sa composition satisfait les conditions suivantes :

$$(\% \, Cr) + 2,5 \, (\% \, Mo) \leq 27 \tag{I}$$

$$(\% \, Cr) + 3,3 \, (\% \, Mo) \geq 26 \tag{II},$$

et

$$LOG \, (\%N) + 0,0605 \, (\%N) = -1,3 + [125 \, (\%V) + 80 \, (\%Nb) + 52$$

$$(\%Cr) + 19 \, (\%Mn)] \times 10^{-3} - [4,3 \, (\%Cr)^2 + 0,35 \, (\%Mn)^2] \times 10^{-4} +$$

$$0,17 \, (\%Cr)^3 \times 10^{-5} \tag{III}.$$

2. Acier suivant la revendication 1 ayant la composition satisfaisant la relation

$$(\%Cr) + 3,3 \, (\%Mo) + 30 \, (\%N) - (\%Mn) \geq 26 \tag{IV}$$

3. Acier selon la revendication 1 ou 2 dont la limite élastique conventionnelle à l'essai de traction Rp 0,2 est supérieure ou égale à 450 N/mm$^2$ après mise en solution à une température supérieure ou égale à 1000°C et hypertrempe.

4. Acier selon l'une des revendications précédente dont la limite élastique conventionnelle à l'essai de traction Rp 0,2 est supérieure ou égale à 1000 N/mm$^2$ et dont l'allongement à la rupture A5d est supérieur à 25% après écrouissage à température ambiante du métal traité par mise en solution à une température supérieure ou égale à 1000°C et hypertrempe.

5.  Utilisation de l'acier selon l'une des revendications précédentes pour la réalisation de pièces destinées à être en contact avec le corps humain en application interne ou externe.

**Patentansprüche**

1.  Nicht-magnetischer rostfreier Stahl mit hohen mechanischen Eigenschaften, resistent gegenüber der Korrosion in physiologischen Milieus und geeignet zur Verarbeitung unter Atmosphärendruck mit der folgenden Zusammensetzung in Gew.%:

| | |
|---|---|
| Mn 15 bis 24 % | Ti $\leq$ 0,020 % |
| Cr 15 bis 20 % | Al $\leq$ 0,020 % |
| Mo 2,5 bis 4 % | S $\leq$ 0,0020 % |
| N 0,6 bis 0,85 % | B $\leq$ 0,020 % |
| V 0,1 bis 0,5 % | Nb+Ta $\leq$ 0,5 % |
| C $\leq$ 0,06 % | Co $\leq$ 0,5 % |
| Ni $\leq$ 0,25 % | Cu $\leq$ 0,5 % |
| Si $\leq$ 0,25 % | |

und 0 bis 0,5 % jeweils der Elemente Cu, Co und der Summe Nb + Ta, wobei der Rest aus Eisen und Verunreinigungen besteht und die Zusammensetzung die folgenden Bedingungen erfüllt:

$$(\%Cr) + 2,5\,(\%Mo) \leq 27 \qquad (I)$$

$$(\%Cr) + 3,3(\%Mo) \geq 26 \qquad (II),$$

und

$$LOG(\%N) + 0,0605(\%N) = -1,3 + [125(\%V) + 80(\%Nb) + 52$$

$$(\%Cr) + 19\,(\%Mn)]x10^{-3} - [4,3(\%Cr)^2 + 0,35(\%Mn)^2]x10^{-4} +$$

$$0,17(\%Cr)^3 x10^{-5} \qquad (III).$$

2.  Stahl nach Anspruch 1 mit der Zusammensetzung, welche die Beziehung

$$(\%Cr) + 3,3(\%Mo) + 30(\%N) - (\%Mn) \geq 26 \qquad (IV)$$

erfüllt.

3.  Stahl nach Anspruch 1 oder 2, dessen konventionelle Elastizitätsgrenze im Zugversuch Rp 0,2 nach dem Inlösungbringen bei einer Temperatur größer oder gleich 1000°C und Überhärtung größer oder gleich 450 N/mm$^2$ ist.

4.  Stahl nach einem der vorhergehenden Ansprüche dessen konventionelle Elastizitätsgrenze im Zugversuch Rp 0,2 größer oder gleich 1000 N/mm$^2$ ist und dessen Bruchdehnung A5d nach dem Nachwalzen bei Umgebungstemperatur des durch Inlösungbringen bei einer Temperatur über oder gleich 1000°C und Überhärtung behandelten Metalles größer 25% ist.

5.  Verwendung des Stahles nach einem der vorhergehenden Ansprüche zur Herstellung von Teilen, die dazu bestimmt sind, mit dem menschlichen Körper bei innerer oder äußerer Anwendung in Kontakt zu sein.

**Claims**

1. A high specification non-magnetic stainless steel which is corrosion resistant in physiological media, and which can be produced at atmospheric pressure
   having the following composition in % by weight:

| Mn 15% to 24% | Ti ≤ 0.020% |
|---|---|
| Cr 15% to 20% | Al ≤ 0.020% |
| Mo 2.5% to 4% | S ≤ 0.0020% |
| N 0.6% to 0.85% | B ≤ 0.020% |
| V 0.1% to 0.5% | Nb + a ≤ 0.5% |
| C ≤ 0.06% | Co ≤ 0.5% |
| Ni ≤ 0.25% | Cu ≤ 0.5% |
| Si ≤ 0.25% | |

and from 0% to 0.5% of each of elements Cu, Co and of the sum Nb + Ta, the remainder being constituted by iron and impurities, and in that its composition satisfies the following conditions:

$$(\%Cr) + 2.5\,(\%Mo) \leq 27 \qquad (I);$$

$$(\%Cr) + 3.3\,(\%Mo) \leq 26 \qquad (II);$$

and

$$LOG\,(\%N) + 0.0605\,(\%N) = -1.3 + [125\,(\%V) + 80\,(\%Nb) + 52\,(\%Cr) + 19$$

$$(\%Mn)] \times 10^{-3} - [\,4.3\,(\%Cr)^2 + 0.35\,(\%Mn)^2\,] \times 10^{-4} + 0.17\,(\%Cr)^3 \times 10^{-5} \qquad (III).$$

2. A steel according to claim 1, having the composition satisfying the relationship:

$$(\%Cr) + 3.3\,(\%Mo) + 30\,(\%N) - (\%Mn) \geq 26 \qquad (IV)$$

3. A steel according to claim 1 or 2 of which the Rp 0.2 tensile test yield strength is 450 N/mm$^2$ or more after solution treatment at a temperature of 1,000°C or more and overhardening.

4. A steel according to one of the preceding claims of which the Rp 0.2 tensile test yield strength is 1,000 N/mm$^2$ and of which the A5d elongation at rupture is more than 25% after ambient temperature work hardening of the metal solution treated at a temperature of 1,000°C or more and overhardening.

5. Use of the steel according to one of the preceding claims to produce parts intended to come into contact with the human body, for internal or external application.